**Europäisches Patentamt**

**European Patent Office**

**Office européen des brevets**

(19)

(11) Numéro de publication : **0 241 357 B1**

(12) **FASCICULE DE BREVET EUROPEEN**

(45) Date de publication du fascicule du brevet :
**11.03.92 Bulletin 92/11**

(51) Int. Cl.⁵ : **C12M 1/00**

(21) Numéro de dépôt : **87400720.6**

(22) Date de dépôt : **01.04.87**

(54) Procédé pour la co-biodégradation différentielle anaérobie d'un substrat cellulosique et d'un substrat propre à la fermentation et appareillage pour la mise en oeuvre de ce procédé.

(30) Priorité : **03.04.86 FR 8604794**

(43) Date de publication de la demande :
**14.10.87 Bulletin 87/42**

(45) Mention de la délivrance du brevet :
**11.03.92 Bulletin 92/11**

(84) Etats contractants désignés :
**BE CH DE ES GB IT LI NL**

(56) Documents cités :
**EP-A- 0 038 759**
**DE-A- 2 535 756**
**FR-A- 914 808**
**FR-A- 1 107 340**
**FR-A- 2 465 696**
**FR-A- 2 480 305**
**FR-A- 2 491 720**

(73) Titulaire : **CENTRE DE COOPERATION INTERNATIONALE EN RECHERCHE AGRONOMIQUE POUR LE DEVELOPPEMENT (C.I.R.A.D.)**
**42 rue Scheffer**
**F-75116 Paris (FR)**

(72) Inventeur : **Forest, Francis**
**74 Avenue de l'Agriculture**
**F-34100 Montpellier (FR)**
Inventeur : **Farinet, Jean-Luc**
**16 rue du Muguet**
**F-34100 Montpellier (FR)**

(74) Mandataire : **Phélip, Bruno et al**
**c/o Cabinet Harlé & Phélip 21, rue de La Rochefoucauld**
**F-75009 Paris (FR)**

**EP 0 241 357 B1**

Jouve, 18, rue Saint-Denis, 75001 PARIS

## Description

La présente invention concerne la fermentation anaérobie d'un substrat cellulosique immergé dans un milieu propre à la fermentation.

Elle a d'abord pour object un procédé pour la bio-dégradation, simultanée, d'une part, d'un substrat propre à la fermentation anaérobie, désigné ci-après par l'expression "substrat propre à la fermentation", et, d'autre part, d'un substrat cellulosique, lesdits substrats progressant dans un fermenteur, pour être extraits, à la sortie de celui-ci, alors qu'ils se trouvent dans un état dégradé. Le substrat cellulosique progresse à vitesse lente dans le fermenteur, dans lequel il est introduit notamment par charges successives. Quant au substrat propre à la fermentation, il progresse à vitesse plus rapide et il est avantageusement introduit en continu dans le fermenteur. Un tel procédé peut être désigné par l'expression "procédé de co-biodégradation différentielle anaérobie" et il conduit à :

– une phase biogaz ;
– une phase solide de faible densité constituée essentiellement par le substrat cellulosique biodégradé ; et
– une phase de consistance liquide à semi-solide constituée par le substrat propre à la fermentation, un fois dégradé.

On peut faire progresser les deux substrats précités dans le fermenteur, soit à co-courant, soit à contre-courant, le sens d'écoulement du substrat propre à la fermentation étant fonction de son degré de pollution et du substrat cellulosique employé. Dans le cas d'un fonctionnement à co-courant, on peut avantageusement effectuer un recyclage du substrat propre à la fermentation, jusqu'à ce que ce dernier soit suffisamment dégradé.

La présente invention a également pour object un appareillage pour la mise en oeuvre de ce procédé.

Le procédé selon la présente invention permet de trai-traiter, comme substrats propres à la fermentation, tous les effluents et sous-produits riches en matières organiques, ayant une consistance allant de liquide à pâteuse et pouvant renfermer jusqu'à un taux élevé de matières en suspension, comme, par exemple, les effluents d'élevage (lisiers) ou d'industries agro-alimentaires, et il permet de faire subir, à ces substrats, une épuration biochimique avant de les évacuer à distance vers des zones d'épandage ou de retraitement sans risque de nuisance, en particulier grâce à la récupération de phase gazeuse durant le temps de transfert de ces substrats dans le fermenteur.

Quant aux substrats cellulosiques, ce sont en particulier les substrats ligno-cellulosiques, d'origine agricole ou ménagère ; ils subissent eux aussi, au cours du procédé, une bio-dégradation, mais, comme indiqué ci-dessus, celle-ci est plus lente.

Les principales techniques de bio-dégradation anaérobie en continu connues à ce jour sont indiquées ci-après :

– on citera d'abord les procédés classiques en continu qui assurent la biodégradation de substrats liquides chargés en matières organiques dans des digesteurs fonctionnant en milieux infiniment mélangés. Ces dispositifs ont l'inconvénient de nécessiter un tamissage préalable et une dilution des matières pour garantir un fonctionnement correct des pompes de circulation. En outre, la biodégradation n'est effectuée qu'avec un faible rendement en raison du fort taux de dilution que ces procédés impliquent.

– des procédés, plus récents et plus perfformants, utilisent la fermentation anaérobie en cellules fixées, sur filtres bactériens, de substrats liquides chargés en matières organiques. Ces dispositifs exigent, pour fonctionner correctement, le respect de consignes: tamisage préalable, interruption du processus en vue d'un décolmatage des filtres, contrôle précis du temps de rétention, démontage des cellules détériorées et leur remplacement. Ces procédés ont le désavantage de ne pas autoriser le traitement d'effluents contenant plus de 10 g/l de matières en suspension.

Dans les deux types de procédés qui viennent d'être évoqués, les règles de maintenance constituent une contrainte pour une mise en oeuvre par des personnes non spécialisées.

Le procédé qui fait l'objet du brevet français n° 2 465 696 ne comporte pas ces inconvénients. Conformément à ce procédé, on introduit, à l'entrée d'un fermenteur de forme allongée et d'axe disposé horizontalement, des charges successives de substrat cellulosique. Chacune des charges introduites, après avoir été immergée dans l'effluent propre à la fermentation, est déplacée vers la sortie du fermenteur, où l'on extrait alors une fraction du substrat cellulosique à l'état dégradé, ainsi que des boues organohumiques. Parallèlement, on recycle les jus de fermentation bactérienne s'écoulant de ces boues organohumiques, à l'aide d'un système de pompage. Il est dont constitué, dans le fermenteur, entre son entrée et sa sortie, une zone de fermentation renfermant une masse de substrat cellulosique immergé dans l'effluent , la partie cellulosique se déplaçant lentement vers la sortie et constituant un lit fixateur des bactéries. On récupère en continu le méthane formé qui s'échappe par des orifices pratiqués à la partie supérieure du fermenteur. Ce procédé permet de traiter des effluents de tous types, liquides à pâteux, sans limitation du taux de matières en suspension.

Pour illustrer l'état antérieur de la technique, on peut également citer l'installation pour la production du méthane par fermentation anaérobie qui est décrite dans la demande de brevet européen n° 0 038 759. Cette installation comprend une cuve pour le traitement des solides et des substrats très concentrés, cette cuve étant munie de cloisons perforées assurant un drainage sensiblement sur toute sa hauteur, maintenant les solides et laissant circuler les liquides et les gaz ; une cuve pour le traitement des substrats dilués qui est équipée d'un serpentin de chauffage ; un dispositif d'introduction des substrats solides pourvu d'un hachoir, d'un tube d'arrivée du jus de recirculation provenant de la cuve de fermentation des substrats liquides et d'un organe d'entrainement ; un dispositif d'introduction des substrats liquides ; un dispositif de recolte du résidu de fermentation provenant de la cuve de fermentation des substrats solides ; et un tube collecteur du methane.

On se trouve ici en présence d'un equipement dans lequel les biodégradations des substrats liquides et solides s'effectuent de façon séparée, d'où il résulte que l'appareillage comporte nécessairement au moins deux cuves, celle destinée au traitement des solides devant être équipée de cloisons perforées. A ceci, vient s'ajouter les dispositifs comme serpentins de réchauffage, hachoir pour la préparation des substrats solides, hélice pour le déplacement de ces derniers, et dispositif de recyclage nécessitant des moyens de pompage. Par ailleurs, le chargement des substrats ne se fait pas à l'air libre et l'évacuation des substrats solides biodégradés fait appel à des moyens strictement mécaniques. La présente invention propose, comme cela ressortira de la decription ci-après, un dispositif de construction beaucoup plus simple et fonctionnant de manière plus efficace que ce dispositif connu.

On connaît également, par la demande de brevet allemand DE-A-2 535 756 une installation pour la production de méthane comportant une cuve cylindrique inclinée munie d'un agitateur rotatif axial. Ce procédé n'est pas susceptible de fonctionner avec une forte densite de chargement et son dispositif pour l'avancement des substrats solides ne permet notamment pas de faire progresser le substrat en continu, d'agiter celui-ci et de briser la croûte formée. La présente invention apporte également une solution à ce problème.

La présente invention apporte un perfectionnement au procédé et à l'appareillage qui sont décrits dans les brevets précités, en premier lieu par le fait qu'elle assure une augmentation du potentiel de fixation des bactéries sur le substrat cellulosique, par suite d'une compression importante exercée sur celui-ci dans le fermenteur, au moment où il pénètre dans la zone dite de fermentation dudit fermenteur. Cette compression augmente la densité dudit substrat, d'où il résulte que le procédé selon la présente invention est, dans son principe, comparable aux filtres bactériens, la particularité étant la mobilité du filtre et sa constitution permettant le traitement à moindre frais de liquides même très chargés en matières en suspension.

Une conséquence intéressante de cette compression est de permettre d'effectuer, dans le cas d'une progression à co-courant des deux substrats mis en oeuvre, le recyclage du substrat propre à la fermentation, sans avoir recours à un moyen de pompage ou à un dispositf accélérateur de circulation. En effet, cette compression entraîne, dans ce cas, une montée du niveau du substrat propre à la fermentation à l'extrémité de sortie du fermenteur, corrélativement à une baisse du niveau de te substrat à l'entrée dudit fermenteur. La différence de pression entre les deux extrémités d'un tube de recyclage, reliant la zone d'entrée et la zone de sortie du fermenteur, provoque un recyclage automatique de ce substrat selon un débit proportionnel au diamètre du tube. La présente invention permet donc d'obtenir, sans aucun risque de colmatage et d'obturation, des débits élevés en substrat propre à la fermentation recyclé, et, par conséquent, des rendements élevés de la biodégradation.

Il suffit, par ailleurs, de modifier la forme de la zone d'extrémité de sortie du fermenteur, pour que la surface libre du substrat propre a la fermentation augmente avec le niveau de ce dernier. Il en résulte une facilité d'évacuation du substrat cellulosique à l'état dégradé, qui vient flotter à la surface de ce substrat propre à la fermentation dans ladite zone d'extrémité de sortie du fermenteur.

La présente invention a donc d'abord pour objet un procédé permettant de réaliser la biodégration simultanée, d'une part, d'une substrat cellulosique, et, d'autre part, d'un substrat propre à la fermentation anaérobie dans lequel ledit substrat cellulosique, à l'état comprimé, est apte à être immergé, procédé suivant lequel on fait progresser, à l'intérieur d'un fermenteur, d'une extrémité à l'autre de celui-ci, chacun des substrats précités, afin de recueillir lesdits substrats, une fois qu'ils ont subi la biodégradation anaérobie, ainsi que le biogaz formé au cours du processus de fermentation, caractérisé par le fait qu'on fait subir au substrat cellulosique, alors qu'ils pénètre dans la zone de fermentation dudit fermenteur, une compression supplémentaire qui conduit à une augmentation de la densité de ce substrat en mouvement lent dans ledit fermenteur, et, de ce fait, à une augmentation de son potentiel de fixation des bactéries.

Conformément à un mode préféré de réalisation du procédé selon la présente invention, on réalise la compression supplémentaire du substrat cellulosique, alors qu'il pénètre dans la zone de fermentation, en le forçant à traverser une zone du fermenteur, dite zone de compression supplémentaire, et de section droite réduite.

De préférence, on choisit comme zone de compression supplémentaire, une zone de forme générale tronconique, se rétrécissant dans le sens du déplacement du substrat cellulosique.

Conformément à un mode particulier de réalisation du procédé selon la présente invention, on introduit le substrat propre à la fermentation anaérobie, en continu, et le substrat cellulosique, de façon séquentielle, en fontion du degré d'épuration recherché.

Conformément a une première variante, on fait progresser le substrat cellulosique et le substrat propre à la fermentation, à co-courant dans le fermenteur, le substrat cellulosique et le substrat propre à la fermentation étant introduits à une extrémité du fermenteur, dans une zone dite d'alimentation , et recueillis, après avoir subi la biodégradation anaérobie, à l'extrémité opposée dudit fermenteur, dans une zone dite zone d'extraction de ce dernier.

Dans le cas d'une progression à co-courant des deux substrats mis en oeuvre, on peut recycler le substrat propre à la fermentation anaérobie et, à cet effet, on fait s'écouler ledit substrat entre la zone d'extraction du fermenteur - lequel présente un axe disposé sensiblement horizontalement - et la zone d'alimentation de ce dernier, la compression du substrat cellulosique dans la zone de compression supplémentaire dudit fermenteur ayant conduit à une montée du niveau du substrat propre à la fermentation dans ladite zone d'extraction, ainsi qu'à une baisse du niveau de ce substrat dans la zone d'alimentation.

On réalise le recylcage du substrat propre à la fermentation notamment à l'aide d'une canalisation reliant directement la partie haute de la zone d'extraction et la zone d'alimentation, ladite canalisation étant, de préférence, inclinée en direction de la zone d'alimentation.

On peut également assurer une régulation de la quantité du substrat propre à la fermentation qui est recyclée, à l'aide d'une vanne de commande située sur la canalisation de recyclage.

Toujours dans le cas d'un fonctionnement à co-courant, on choisit avantageusement une zone d'extraction dans laquelle la surface libre du substrat propre à la fermentation augmente avec le niveau dudit substrat.

Conformément à une caractéristique particulièrement intéressante du procédé selon l'invention, on évacue par trop plein le résidu cellulosique flottant à la surface du, ou immergé dans, le substrat propre à la fermentation anaérobie dans la zone d'extraction du fermenteur, en déplaçant ledit résidu vers l'extérieur dans la direction horizontale ou dans la direction verticale, à l'aide d'un moyen mécanique, l'évacuation pouvant également s'effectuer par trop-plein, le cas échéant à l'aide d'un moyen mécanique déplaçant le résidu.

Par ailleurs, comme indiqué ci-dessous, on peut également faire progresser le substrat cellulosique et le substrat propre à la fermentation à contre-courant dans le fermenteur.

Conformément à d'autres caractéristiques du procédé selon la présente invention, on réalise la compression d'une charge de substrat cellulosique, après introduction dans la zone d'alimentation mais avant déplacement vers la zone de fermentation, en faisant peser sur ladite charge, la charge suivante en attente dans une zone de chargement du fermenteur et bloquée dans ladite zone de chargement ; et on commande à distance le chargement des substrats, l'avancement des charges de substrat cellulosique, le recyclage du substrat propre à la fermentation, l'extraction du résidu cellulosique et le sou tirage du substrat propre à la fermentation, en fonction de données concernant la composition du substrat propre à la fermentation qui se trouve dans la zone d'extraction.

La présente invention a également pour objet une appareillage pour la mise en oeuvre du procédé qui vient d'être défini, ledit appareillage comportant :

– un fermenteur ;

– un moyen pour introduire, dans ledit fermenteur, à une extrémité de celui-ci, le substrat cellulosique qui doit subir une dégradation ;

– un moyen pour assurer la compression dudit substrat cellulosique pour qu'il entre,à l'état comprimé, dans ledit fermenteur ;

– un dispositif pour faire avancer, dans ledit fermenteur, le substrat cellulosique introduit à une extrémité, en direction de l'extrémite opposée ;

– un moyen pour assurer la compression supplémentaire du substrat cellulosique introduit dans ledit fermenteur, alors qu'il entre dans la zone de fermentation de ce dernier ;

– un moyen pour introduire, dans ledit fermenteur, à une extrémité de celui-ci, le substrat cellulosique qui doit lui aussi subir une dégradation et pour le faire s'écouler en direction de l'extrémité opposée ;

– un collecteur de biogaz disposé à la partie supérieure dudit fermenteur, au-dessus de la zone de fermentation ;

– une canalisation de soutirage du substrat propre à la fermentation, à l'état biodégradé ; et

– un dispositif d'extraction du substrat cellulosique à l'état biodégradé.

Conformément à un mode particulier de réalisation de l'appareillage selon la présente invention, le fermenteur présente, à l'entrée de la zone de fermentation, une section droite plus étroite, afin de constituer la zone de compression supplémentaire, le rétrécissement de cette section droit n'étant éventuellement pas pratiqué dans la zone inférieure dudit fermenteur. De préférence, cette zone de compression supplémentaire est une zone de forme d'ensemble tronconique se rétrécissant en direction de la zone de fermentation.

S'agissant d'un fermenteur dans lequel le substrat cellulosique et le substrat propre à la fermentation sont susceptibles de progresser à co-courant, le fermenteur, qui présente une forme allongée et dont l'axe est disposé sensiblement horizontalement, comporte successivement, d'une extrémité à l'autre, une zone d'alimentation, la zone de fermentation et une zone d'extraction, deux ouvertures étant prévues dans la zone d'alimentation pour l'introduction des charges respectivement de substrat cellulosique et de substrat propre à la fermentation, ledit appareillage étant, le cas échéant, doté d'une canalisation de recyclage par simple écoulement des jus de fermentation depuis la zone d'extraction vers la zone d'alimentation, sur laquelle est éventuellement disposée une vanne de commande de la régulation dudit recyclage.

Conformément à une caractéristique particulière, la zone d'extraction est délimitée par un bac comportant une paroi verticale, dans laquelle débouche perpendiculairement la paroi du fermenteur délimitant la zone de fermentation, la canalisation de recyclage étant reliée à ladite paroi verticale, dans sa partie située au-dessus de la zone de fermentation.

Par ailleurs, ce bac présente avantageusement une forme évasée en direction de sa partie supérieure.

L'appareillage selon la présente invention peut également comporter avantageusement, au-dessus de la zone d'extraction, un dispositif permettant de déplacer vers l'extérieur, dans la direction horizontale ou dans la direction verticale, le résidu cellulosique biodégradé flottant à la surface du, ou immergé dans le substrat propre à la fermentation.

En outre, le dispositif pour faire avancer le substrat cellulosique dans le fermenteur, comprend, d'une part, au moins un arbre traversant ledit fermenteur dans la direction longitudinale, parallèlement à son axe, et susceptible d'être animé d'un movement de va-et-vient ; ledit (ou lesdits) arbre(s) comportant des dents permettant un accrochage du substrat cellulosique dans le sens de la poussée et une désolidarisation dudit susbtrat dans un sens opposée, et, d'autre part, une organe de poussée dudit substrat, introduit par charges successives, qui est solidaire dudit (ou desdits) arbre(s) denté(s).

Par ailleurs, l'appareillage selon la présente invention peut également comporter une rampe verticale de chargement du substrat cellulosique suivant des charges successives, ladite rampe comportant un doigt de retenue des charges avant leur introduction dans la zone d'alimentation .

L'appareillage selon l'invention peut également comprendre un dispositif de commande à distance du chargement des substrats, de l'avancement du substrat cellulosique, du recyclage du substrat propre à la fermentation, de l'extraction du résidu cellulosique et du soutirage du substrat propre à la fermentation, en fonction de données qui concernent le substrat propre à là fermentation et qui proviennent d'un élément détectant le niveau de pollution et se trouvant dans la zone d'extraction.

Pour mieux faire comprendre l'objet de la présente invention, on décrira plus en détail ci-après, à titre indicatif et non limitatif, un mode particulier de réalisation d'un appareillage pour la mise en oeuvre du procédé selon la présente invention, dans lequel les deux substrats mis en jeu progressent à co-courant dans le fermenteur, ce en référence au dessin annexé.

Sur ce dessin :
– la figure 1 est une vue schématique de cet appareillage en cours de fonctionnement, le récipient de fermentation étant représenté partiellement en coupe verticale longitudinale effectuée selon l'axe dudit récipient ;
– la figure 2 est une vue partielle en coupe axiale verticale dudit récipient ;
– la figure 3 est une vue partielle en coupe horizontale, selon III-III de la figure 2 ;
– la figure 4 est une vue en coupe verticale transversale selon IV-IV de la figure 2 ;
– la figure 5 représente, à plus grande échelle, le détail D de la figure 4.

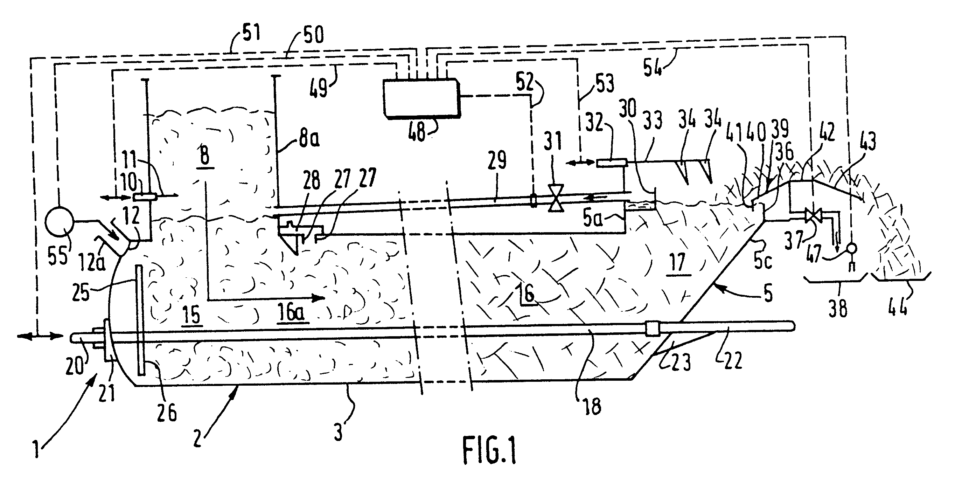

Si l'on se réfère à la figure 1 du dessin annexé, on voit que l'on a représenté par 1 dans son ensemble, un appareillage pour la biodégradation en continu d'un effluent pollué sous l'action de bactéries anaérobies fixées sur un lit ligno-cellulosique lui-même biodégradable, mobile, constitué à partir de charges successives d'un substrat ligno-cellulosique, ledit effluent et ledit lit ligno-cellulosique se déplaçant dans la même direction.

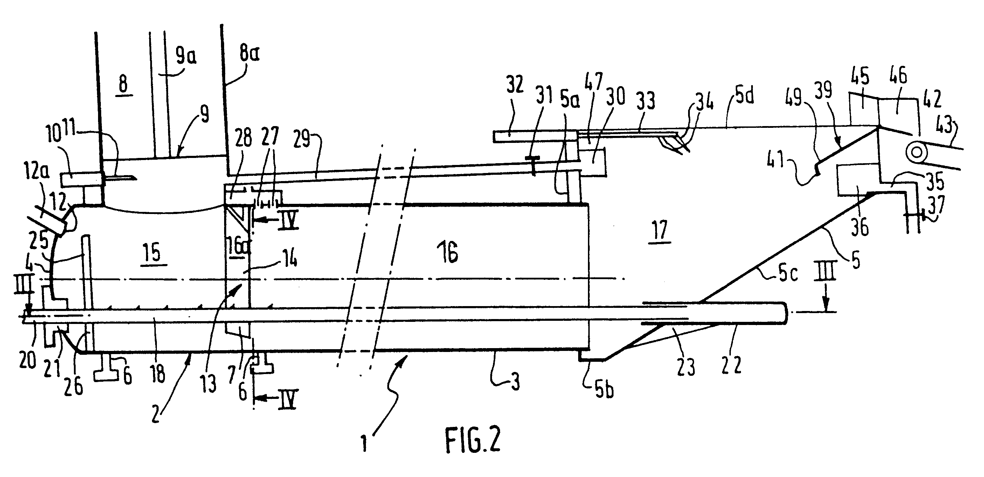

Comme on peut le voir également sur la figure 2, l'appareillage 1 est constitué par un fermenteur 2 et par ses équipements annexes. Le fermenteur 2 comporte une paroi cylindrique 3, qui n'est représentée que partiellement sur le dessin, et dont l'axe est disposé incliné de 3 à 6°, notamment de 4° vers le sol depuis son extrémité d'entrée vers son extrémite de sortie. Ladite paroi 3 est fermée à l'une de ses extrémités par une paroi 4 en forme de calotte sphérique et de concavité dirigée vers l'intérieur de la paroi 3. L'extrémité libre de cette dernière débouche dans la paroi verticale 5a d'un bac 5 ouvert à sa partie supérieure et délimité, en dehors de la paroi 5a qui est disposée perpendiculairement à l'axe de la paroi 3 par une paroi de fond horizontale 5b, par une paroi latérale 5c s'écartant progressivement de la paroi 5a depuis la paroi de fond 5b, puis redevenant parallèle à ladite paroi 5a, et par deux autres parois verticales opposées 5d, réunissant les parois 5a et 5c.

Le fermenteur 2 repose sur le sol par la paroi 5b du bac 5 et par l'intermédiaire de plusieurs pieds 6, régulièrement espacés, portés par la paroi cylindrique 3.

A sa partie supérieure et au voisinage de la paroi d'extrémité 4, la paroi cylindrique 3 comporte une ouverture 7 bordée extérieurement par une paroi tubulaire 8a qui présente un axe perpendiculaire à celui de la paroi cylindrique 3 et qui constitue une rampe verticale de chargement. Dans cette paroi 8a, qui délimite une zone de chargement 8, est monté déplaçable dans la direction verticale, un piston 9 dont la tige 9a est reliée à un treuil ou à un vérin hydraulique non représenté sur le dessin.

La paroi tubulaire 8 est, par ailleurs, traversée, du côté de la paroi d'extrémité 4 et légèrement au-dessus de cette dernière, par un manchon 10, dans lequel est susceptible de se déplacer, suivant un mouvement de va-et-vient dans la direction horizontale, un doigt 11 qui, dans la position où il est complètement en saillie vers l'intérieur de la zone de chargement 8, assure la retenue d'un bloc de substrat ligno-cellulosique.

La paroi d'extrémité 4 comporte, dans sa partie supérieure, une ouverture 12 bordée extérieurement par une tubulure 12a inclinée en direction de l'axe de la paroi cylindrique 3, cette tubulure 12a permettant l'introduction, dans le fermenteur 2, de l'effluent à biodégrader.

La paroi tubulaire 3 présente, en outre, immédiatement après l'ouverture 7, dans la direction opposée à la paroi d'extrémité 4, et sauf dans sa région inférieure, un rétrécissement de sa section droite, sur une courte distance. A cet effet, ladite paroi 3 porte intérieurement un élément annulaire 13, ouvert à sa partie inférieure pour la raison indiquée ci-après, présentant une paroi tronconique 14, dont l'axe est confondu avec celui de la paroi 3 et dont la bordure de plus grande section est reliée à ladite paroi 3.

La région 15 du fermenteur 2, située entre la paroi d'extrémité 4 et l'élément annulaire 13, constitue la zone d'alimentation de l'effluent à biodégrader et des charges de substrat ligno-cellulosique. Quant à la région 16 dudit fermenteur 2 située entre l'élément annulaire 13 et l'extrémité ouverte de la paroi 3, elle constitue la zone de fermentation proprement dite, dont l'entrée 16a représente la zone de compression du substrat cellulosique introduit. Quant au bac 5, il délimite une zone 17, dite zone d'extraction.

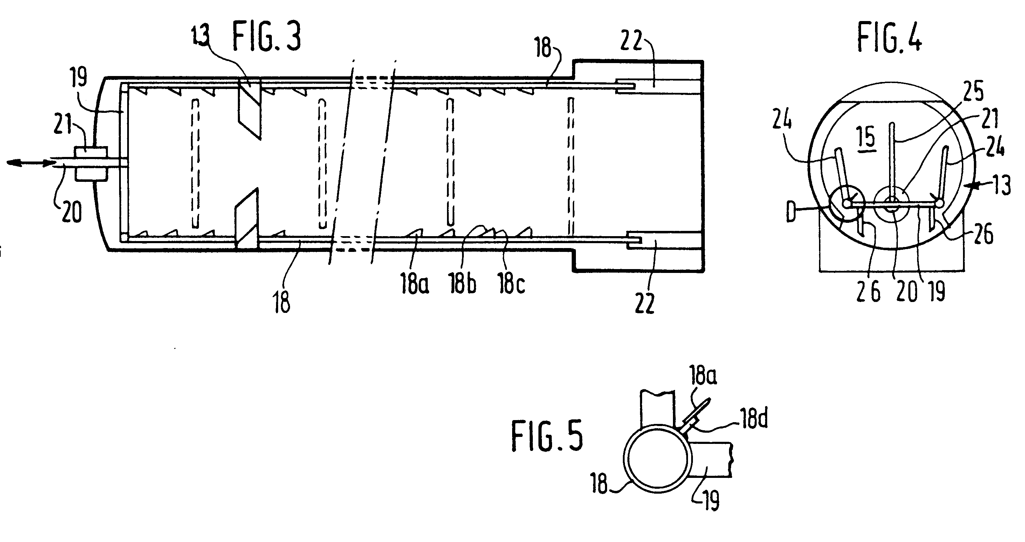

Le fermenteur 2 est traversé, dans un même plan sensiblement horizontal situé au-dessous de l'axe de la paroi cylindrique 3, par deux arbres 18, disposés parallèlement audit axe, situés chacun à proximité de la paroi 3, et à égale distante de cette paroi. Comme on peut le voir notamment sur la figure 3, chaque arbre 18 porte des dents 18a régulièrement espacées, dirigées vers l'intérieur de la paroi 3, chaque dent 18a comportant une face inclinées 18b s'écartant de l'arbre 18 dans la direction opposée à la paroi de fond 4, et une face 18c perpendiculaire audit arbre 18. Chaque dent 18a est portée par un support de tenture 18d lié à l'arbre 18 associé.

Les dents 18a sont de forme et dimension variables suivant la nature du substrat cellulosique, elles sont ici représentées de façon schématique.

A leur extrémité située du côté de la paroi 4, les deux arbres 18 sont réunis par une tige horizontale 19, au centre de laquelle est relié un bras 20 traversant perpendiculairement la paroi 4 avec interposition d'un joint d'étanchéité 21 fixé à cette dernière, le bras 20 est connecté, par son extrémité émergeant du joint d'étanchéité 21, à l'extérieur du fermenteur 2, à un vérin hydraulique, ou autre dispositif de poussée, non représenté sur le dessin.

Chaque arbre 18 est guidé, à son extrémité opposée, dans un fourreau horizontal 22 traversant la paroi 5c du bac 5. Une nervure 23 (figure 2), disposée sous chaque fourreau 22, relie extérieurement ce dernier avec la paroi 5c.

Par ailleurs, dans le plan transversal du fermenteur 2 contenant la tige 19, sont disposées trois barres dirigées vers la partie supérieure de la zone 15, à savoir deux barres latérales 24, chacune étant reliée à une extrémité de la tige 19, et une barre centrale 25. De plus, la tige 19 porte, dans ce même plan, deux barres racleuses plus courtes 26 dirigées dans la zone inférieure de la zone 15 et perpendiculairement à la tige 19. L'ensemble des barres 24 à 26 constitue un organe de poussée de la charge qui sera introduite dans la zone d'alimentation 15. L'élément 13 est ouvert vers le bas pour autoriser le passage des barres racleuses 26 lors de l'avance de cet organe de poussée. En position de repos, celui-ci se situe en retrait, par rapport à l'ouverture 7, du côté de la paroi d'extrémité 4.

En fonction de la nature du substrat cellulosique à traiter, les deux arbres 18 seront plus ou moins rapprochés, nécessitant ou non l'ouverture vers le bas de l'élément 13.

La paroi cylindrique 3 comporte, en outre, à sa partie supérieure, au voisinage de l'élément annulaire 13, du côté de la zone de fermentation 16, plusieurs orifices 27 pour l'évacuation du biogaz formé au cours de la fermentation, ces orifices 27 débouchant dans un collecteur de biogaz 28.

La paroi 5a du bac 5 est traversée, à sa partie supérieure, au-dessus de la paroi 3, par une canalisation de recyclage 29 qui débouche à l'intérieur dudit bac 5, dans un déversoir 30. Ladite canalisation de recyclage 29, sur laquelle est disposée une vanne 31, relie la partie supérieure de la zone 17 à la zone de chargement 8, et elle est légèrement inclinée vers le bas en direction de la paroi d'extrémité 4 du fermenteur 2.

La paroi 5a comporte également, le long de sa bordure supérieure, un manchon 32, qui est disposé parallèlement à l'axe de la paroi cylindrique 3 et dans lequel est montée coulissante une tige 33 surplombant la zone d'extraction 17 et portant, à son extrémité libre, plusieurs griffes 34 dirigées vers le bas ; le dispositif constitué par la tige 33 et les griffes 34 sert à faire glisser vers la sortie le résidu cellulosique dégradé qui flotte en surface du substrat propre à la fermentation dans la zone 17. On pourrait aussi envisager le montage d'un tapis évacuateur, immergé dans la zone 17.

La paroi 5c, dans la zone de jonction entre sa partie inférieure inclinée et sa partie supérieure verticale, est traversée par une conduite 35 située à l'extérieur, cette conduite 35 débouchant dans un déversoir 36 intérieur au bac 5. Cette conduite 35, sur laquelle est disposée une vanne 37, est coudée vers le bas et elle permettra l'évacuation du substrat propre à la fermentation considéré comme suffisamment dégradé, dans un bac récepteur 38 (figure 1).

Le bac 5 comporte également une rampe 39 pour favoriser l'évacuation du résidu cellulosique dégradé, cette rampe 39 se situant à l'opposé de la zone de fermentation 16. La rampe 39 comporte une partie 40 disposée à l'intérieur du bac 5 et présentant une extrémité libre 41 recourbée, et une partie 4 extérieure au bac 5. La partie 40-41, d'inclinaison sensiblement analogue à celle de la paroi 5c, empêche les résidus cellulosiques de pénétrer dans le déversoir 36 ; quant à la partie 42, inclinée dans le sens opposé, elle permet de guider les résidus cellulosiques sortant du bac 5 vers un transporteur 43, les conduisant dans un bac récepteur 44 (figure 1).

Au niveau de la rampe 39, le bac est équipé de plaques verticales 45 et 46 assurant le guidage des résidus vers le transporteur 43.

L'appareillage 1 comporte également un élément 47 destiné à détecter le niveau de pollution, notamment une sonde, par exemple, à oxygène, disposée à l'intérieur du bac 5, au-dessus du réservoir 30.

De plus, comme cela est schématisé sur la figure 1, l'appareillage 1 peut comporter un dispositif 48 de commande à distance de différentes opérations du procédé, ce dispositif 48 étant relié aux dispositifs de commande respectivement du doigt de retenue 11 prévu sur la rampe de chargement 8a, de l'introduction de l'effluent à biodégrader par la tubulure 12a, du mouvement d'avance et de recul des arbres 18, de la vanne 31 de la canalisation 29 de recyclage, du dispositif 33-34 aidant à l'extraction du substrat cellulosique dégradé, et de la vanne 37 de soutirage de l'effluent dégradé. Sur la figure 1, les connexions entre le dispositif 48 et les diférents dispositifs de commande qui viennent d'être indiqués ont été repérés par le chiffres de référence respectivement 49 à 54.

Sur la figure 1, on a montré une charge cellulosique en attente dans la zone de chargement 8, cette charge étant retenue par le doigt 11 ; une charge cellulosique prête à être introduite dans la zone de fermentation 16 est également montrée, occupant la zone d'alimentation 15. Quant à la zone 16, elle est occupée par le substrat cellulosique immergé dans l'effluent à biodégrader, le substrat cellulosique biodégradé étant évacué par-dessus la rampe 39 et tombant dans le bac 44.

Le mouvement des charges cellulosiques à l'intérieur du fermenteur 2 est le suivant :

– lorsque l'on désire introduire, dans la zone de fermentation 16, la charge cellulosique qui se trouve dans la zone d'alimentation 15, on commande l'avance des arbres 18 en alimentant le vérin connecté à la tige 20. La charge cellulosique en question pénètre dans la zone 16, en subissant, dans la zone 16a, une compression. Dans ces conditions, toute la masse en fermentation occupant la zone 16 se déplace lentement en direction de la zone d'extraction 17.

– lorsque l'on désire introduire une nouvelle charge, on commande le retrait des arbres 18 (la masse cellulosique en travail n'étant pas entraînée vers l'arrière vu la forme des dents 18a), puis le retrait du doigt de retenue 11, ce qui permet à la charge en attente dans la zone de chargement 8, de venir se placer dans la zone d'alimentation 15. On introduit alors la nouvelle charge de substrat cellulosique dans la zone 8, à l'aide du piston 9 qui vient en appui sur elle, et on s'arrange pour que cette nouvelle charge soit retenue par le doigt 11. La nouvelle charge pèse sur la charge qui se trouve dans la zone 15 et qui est prête à être introduite dans la zone de fermentation 16, ce qui permet de comprimer cette dernière charge avant son entrée dans la zone de fermentation 16.

Le mouvement de l'effluent à biodégrader est le suivant :

– en fonctionnement normal (c'est-à-dire sans recyclage), on introduit ledit effluent dans un fermenteur, d'une manière continue, par pompage à l'aide de la pompe 55 (figure 1) dans la zone 15 dudit fermenteur 2, par la tubulure 12a. L'effluent imprègne alors les charges cellulosiques qui se trouvent dans le fermenteur 2. Dans la zone d'extraction 17, compte tenu de la forme particulière du bac 5, le résidu cellulosique à l'état biodégradé, vient flotter en surface de l'effluent à dégrader se trouvant alors dans ledit bac 5 et peut être évacué en passant sur la rampe 39. On peut aider, à cette évacuation, en commandant la manoeuvre du dispositif 33-34.

Pour récupérer l'effluent ayant subi la biodégradation, on ouvre la vanne 37.

Par ailleurs, on récupère en continu le méthane et le gaz carbonique qui s'échappent par les orifices 27 pour pénétrer dans le collector 28.

Si toutefois, on estime que l'effluent n'est pas suffisamment dégradé, renseignements fournis par l'élément détecteur 47, on le fait recirculer dans le fermenteur 2 en procédant de la façon suivante :

– on arrête l'alimentation en effluent dans la zone d'alimentation 15 ;

– on ferme la vanne 37 et on ouvre la vanne 31 ; et

– on introduit une charge de substrat cellulosique, ce qui provoque le recyclage automatique qui s'explique comme suit :

Par suite de la compression exercée sur ladite charge dans la zone 16a, le niveau de l'effluent à dégrader s'élève dans la zone d'extraction 17. Simultanément, le niveau dudit effluent baisse dans la zone d'alimentation, si bien que ledit effluent peut s'écouler automatiquement à travers la canalisation 29 sur laquelle la vanne 31 a été ouverte.

On constate donc que le recyclage a lieu sans qu'il ne soit nécessaire de recourir à un moyen de pompage.

Il est bien entendu que le mode de réalisation ci-dessus décrit n'est aucunement limitatif et pourra donner lieu à toutes modifications désirables, sans sortir pour cela du cadre de l'invention.

On pourra ainsi prévoir que le (ou les) arbres(s) 18 du dispositif destiné à faire avancer le substrat cellulosique dans le fermenteur 2 soient des arbres creux logeant un dispositif de réchauffage pouvant, si on le désire, assurer le réchauffage du substrat au coeur du fermenteur. Il est donc intéressant de souligner cet avantage qui s'ajoute au fait que ce dispositif destiné à faire avancer le substrat, animé d'un mouvement alternatif, assure une agitation du substrat, le bris des croûtes, et un dégazage pendant les courses de retour par vibration et frottement le long de la paroi des arbres 18 munie des dents 18a.

De plus, le dispositif de la présente invention offre la possibilité de travailler avec des sustrats progressant alternativement à co-courant et à contre-courant, ce qui permet d'éviter les opérations de décolmatage obligeant au démontage du fermenteur.

## Revendications

1. Procédé permettant de réaliser la biodégradation simultanée, d'une part, d'un substrat cellulosique, et, d'autre part, d'un substrat propre à la fermentation anaérobie dans lequel ledit substrat cellulosique, à l'état comprimé, est apte à être immergé, procédé suivant lequel on fait progresser à l'intérieur d'un fermenteur (2), d'une extrémité à l'autre de celui-ci, chacun des substrats précités, afin de recueillir lesdits substrats, une fois qu'ils ont subi la biodégradation anaérobie, ainsi que le bioagaz formé au cours de processus de fermentation, caractérisé par le fait qu'on fait subir au substrat cellulosique, alors qu'il pénètre à l'état comprimé dans la zone de fermentation (16) dudit fermenteur (2) une compression supplémentaire qui conduit à une augmentation de la densité de ce substrat en mouvement lent dans ledit fermenteur (2), et, de ce fait, à une augmentation de son potentiel de fixation des bactéries.

2. Procédé selon la revendication 1, caractérisé par le fait qu'on réalise la compression supplémentaire du substrat cellulosique, alors qu'il pénètre dans la zone de fermentation (16), en le forçant à traverser une zone du fermenteur (2), dite zone de compression supplémentaire (16a), de section droite réduite.

3. Procédé selon la revendication 2, caractérisé par le fait qu'on choisit, comme zone de compression supplémentaire (16a), une zone de forme générale tronconique, se rétrécissant dans le sens de déplacement du substrat cellulosique.

4. Procédé selon l'une des revendications 1 à 3, caractérisé par le fait qu'on introduit le substrat propre à la fermentation anaérobie, en continu, et le substrat cellulosique, de façon séquentielle, en fonction du degré d'épuration recherché.

5. Procédé selon l'une des revendication 1 à 4, caractérisé par le fait qu'on fait progresser le substrat cellulosique et le substrat propre à la fermentation anaérobie à co-courant dans le fermenteur (2), le substrat cellulosique et le substrat propre à la fermentation anaérobie étant introduits à une extrémité du fermenteur (2), dans une zone dite zone d'alimentation (15), et recueillis, après avoir subi la biodégradation anaérobie, à l'extémité opposée dudit fermenteur (2), dans une zone dite zone d'extraction (17) de ce dernier.

6. Procédé selon la revendication 5, dans lequel ledit fermenteur (2) présente un axe disposé sensiblement horizontalement, caractérisé par le fait qu'on recycle le substrat propre à la fermentation, la compression du substrat cellulosique dans la zone de compression supplémentaire (16a) dudit fermenteur (2) conduisant à une montée du niveau du substrat propre à la fermentation anaérobie dans ladite zone d'extraction (17) ainsi qu'à une baisse du niveau de ce substrat dans la zone d'alimentation (15), permettant ainsi un recyclage sans moyen de pompage.

7. Procédé selon la revendication 6, caractérisé par le fait qu'on réalise le recyclage du substrat propre à la fermentation anaérobie à l'aide d'une canalisation (29) reliant directement la partie haute de la zone d'extraction (17) et la zone d'alimentation (15), ladite canalisation (29) étant, de préférence, inclinée en direction de la zone d'alimentation (15).

8. Procédé selon la revendication 7, caractérisé par le fait qu'on assure une régulation de la quantité du substrat propre à la fermentation anaérobie qui est recyclée, à l'aide d'une vanne de commande (31) située sur la canalisation de recyclage (29).

9. Procédé selon l'une des revendications 5 à 8, caractérisé par le fait qu'on choisit une zone d'extraction (17) dans laquelle la surface libre du substrat propre à la fermentation anaérobie augmente avec le niveau dudit substrat.

10. Procédé selon l'une des revendications 5 à 9, caractérisé par le fait qu'on évacue le résidu cellulosique flottant à la surface du, ou immergé dans le, substrat propre à la fermentation anaérobie dans la zone d'extraction (17) en déplaçant ledit résidu vers l'extérieur dans la direction horizonle ou dans la direction verticale, à l'aide d'un moyen mécanique, l'évacuation pouvant également s'effectuer par trop-plein, le cas échéant avec l'aide d'un moyen mécanique déplaçant le résidu.

11. Procédé selon l'une des revendications 1 à 4, caractérisé par le fait qu'on fait progresser le substrat cellulosique et le substrat propre à la fermentation anaérobie à contre-courant dans le fermenteur.

12. Procédé selon l'une des revendications 1 à 11, caractérisé par le fait qu'on réalise la compression d'une charge de substrat cellulosique, après introduction dans la zone d'alimentation (15) mais avant déplacement vers la zone de fermentation (16), en faisant peser sur ladite charge, la charge suivante en attente dans une zone de chargement (8) du fermenteur (2) et bloquée dans ladite zone de chargement (8).

13. Procédé selon les revendications 1,4,5,6,8,9 et 10, prises simultanément, caractérisé par le fait qu'on commande à distance le chargement de substrats, l'avancement des charges de substrat cellulosique, le recyclage du substrat propre à la fermentation anaérobie, l'extraction du résidu cellulosique et le soutirage du substrat propre à la fermentation anaérobie, ayant subi la dégradation voulue, en fonction de données concernant la composition du substrat propre à la fermentation anaérobie qui se trouve dans la zone d'extraction (17).

14. Appareillage pour la mise en oeuvre du procédé tel que défini à l'une des revendications 1 à 13, caractérisé par le fait qu'il comporte :
– un fermenteur (2) ;
– un moyen pour introduire, dans ledit fermenteur (2), à une extrémité de celui-ci, le substrat cellulosique, qui doit subir une dégradation ;
– un moyen pour assurer la compression dudit substrat cellulosique pour qu'il entre à l'état comprimé, dans ledit fermenteur (2) ;
– un dispositif pour faire avancer, dans ledit fermenteur (2), le substrat cellulosique introduit à une extrémité, en direction de l'extrémité opposée ;
– un moyen pour assurer la compression supplémentaire du substrat cellulosique introduit dans ledit fermenteur (2), alors qu'il entre dans la zone de fermentation (16) dudit fermenteur (2) ;
– un moyen pour introduire, dans ledit fermenteur (2), à une extrémité de celui-ci, le substrat cellulosique qui doit lui aussi subir une dégradation et pour le faire s'écouler en direction de l'extrémité opposée ;
– un collecteur de biogaz (28) disposé à la partie supérieure dudit fermenteur (2), au-dessus de la zone de fermentation (16) ;
– une canalisation (35) de soutirage du substrat propre à la fermentation, à l'état biodégradé ; et
– un dispositif d'extraction du substrat cellulosique à l'état biodégradé.

15. Appareillage selon la revendication 14, caractérisé par le fait que le fermenteur (2) présente, à l'entrée de la zone de fermentation (16), une section droite plus étroite, afin de constituer la zone de compression supplémentaire (16a), le rétrécissement de cette section droite n'étant éventuellement pas pratiqué dans la zone inférieure dudit fermenteur (2).

16. Appareillage selon la revendication 15, caractérisé par le fait que la zone de compression supplémentaire (16a) est une zone de forme d'ensemble tronconique se rétrécissant en direction de la zone de fermentation (16).

17. Appareillage selon l'une des revendications 14 à 16, comportant un fermenteur (2) dans lequel le substrat cellulosique et le substrat propre à la fermentation anaérobie sont susceptibles de progresser à co-courant, caractérisé par le fait que le fermenteur (2) qui présente une forme allongée et dont l'axe est disposé sensiblement horizontalement, comporte successivement, d'une extrémité à l'autre, une zone d'alimentation (15), la zone de fermentation (16) et une zone d'extraction (17), deux ouvertures (7 ; 12) étant prévues dans la zone d'alimentation (15) pour l'introduction des charges respectivement de substrat cellulosique et de substrat propre à la fermentation anaérobie, ledit appareillage (1) étant, le cas échéant, doté d'une canalisation (29) de recyclage par simple écoulement des jus de fermentation, depuis la zone d'extraction (17) vers la zone d'alimentation (15), sur laquelle est éventuellement disposée une vanne (31) de commande de la régulation dudit recyclage.

18. Appareillage selon la revendication 17, caractérisé par le fait que la zone d'extraction (17) est délimitée par un bac (5) comportant une paroi verticale (5a), dans laquelle débouche perpendiculairement la paroi (3) du fermenteur (2) délimitant la zone de fermentation (16), la canalisation de recyclage (29) étant reliée à ladite paroi verticale (5a), dans sa partie située au-dessus de la zone de fermentation (16).

19. Appareillage selon la revendication 18, caractérisé par le fait que le bac (5) présente une forme évasée en direction de sa partie supérieure.

20. Appareillage selon l'une des revendications 17 à 19, caractérisé par le fait qu'il comporte, au-dessus de la zone d'extraction (17), un dispositif (33) permettant de déplacer vers l'extérieur, dans la direction horizontale ou dans la direction verticale, le résidu cellulosique biodégradé flottant à la surface du ou immergé dans le substrat propre à la fermentation.

21. Appareillage selon l'une des revendications 14 à 20, caractérisé par le fait que le dispositif pour faire avancer le substrat cellulosique dans le fermenteur (2), comprend, d'une part, au moins un arbre (18) traversant ledit fermenteur (2) dans la direction longitudinale, parallèlement à son axe, et susceptible d'être animé d'un mouvement de va-et-vient ; ledit (ou lesdits) arbre(s) (18) comportant des dents (18a) permettant un accrochage du substrat cellulosique dans le sens de la poussée et une désolidarisation dudit substrat dans un sens opposé, et, d'autre part, un organe de poussée (24 à 26) dudit substrat, introduit par charges successives, solidaire dudit (ou desdits) arbre(s) denté(s) (18).

22. Appareillage selon l'une des revendications 14 à 21, caractérisé par le fait qu'il comporte une rampe verticale (8a) de chargement du substrat cellulosique suivant des charges successives, ladite rampe (8a) comportant un doigt de retenue (11) des charges avant leur introduction dans la zone d'alimentation (15).

23. Appareillage selon l'une des revendications 20 à 22, caractérisé par le fait qu'il comprend un dispositif (48) de commande à distance du chargement des substrats, de l'avancement du substrat cellulosique, du recyclage du substrat propre à la fermentation, de l'extraction du résidu cellulosique et du soutirage du substrat propre à la fermentation, en fonction de données qui concernent le substrat propre à la fermentation et proviennent d'un élément (47) détectant le niveau de pollution et se trouvant dans la zone d'extraction (17).

24. Appareillage selon l'une des revendications 21 à 23, caractérisé par le fait que le (ou les) arbre(s) (18) du dispositif pour faire avancer le substrat cellulosique dans le fermenteur (2) sont des arbres creux, logeant des moyens de réchauffage dudit substrat au sein du fermenteur.

**Patentansprüche**

1. Verfahren zum simultanen Bioabbau einerseits eines zellulosehaltigen Trägermaterials und andererseits eines für die anaerobe Fermentierung geeigneten Trägermaterials, in welches das zellulosehaltige Trägermaterial komprimiert eingetaucht werden kann, wobei nach dem Verfahren jedes der genannten Trägermaterialien durch einen Fermentierer (2) von dessen einem Ende zum anderen Ende fortgeleitet wird, damit die Trägermaterialien, nachdem sie dem Bioabbau unterlagen, ebenso das im Verlaufe des Fermentierungsvorgangs gebildete Biogas, gesammelt werden, **dadurch gekennzeichnet**, daß auf das zellulosehaltige Trägermaterial, sobald es in komprimiertem Zustand in die Fermentierungszone (16) des Fermentierers (2) eindringt, eine Zusatzverdichtung ausgeübt wird, die bei langsamer Bewegung des Trägermaterials in dem Fermentierer zu einer Dichteerhöhung führt und daher zu einem verstärkten Anhaftungspotential von Bakterien.

2. Verfahren nach Anspruch 1, **dadurch gekennzeichnet**, daß die Zusatzverdichtung des zellulosehaltigen Trägermaterials dann erfolgt, wenn es in die Fermentierungszone (16) eindringt, indem es zum Durchgang durch eine Zone des Fermentierers (2), nämlich eine Zone zusätzlicher Verdichtung (16a), mit reduziertem querschnitt gezwungen wird.

3. Verfahren nach einem der Anspruch 2, **dadurch gekennzeichnet**, daß als Zone zusätzlicher Verdichtung (16a) eine Zone ausgewählt wird, die eine im allgemeinen kegelstumpfförmige Gestalt aufweist, die sich im Sinne der Verlagerung des zellulosehaltigen Trägermaterials verjüngt.

4. Verfahren nach einem der Ansprüche 1 - 3, **dadurch gekennzeichnet**, daß das für die anaerobe Fermentierung geeignete Trägermaterial fortlaufend eingeführt wird und das zellulosehaltige Trägermaterial sequentiell, abhängig von dem gewünschten Reinigungsgrad, eingeführt wird.

5. Verfahren nach einem der Ansprüche 1 - 4, **dadurch gekennzeichnet**, daß das zellulosehaltige Trägermaterial und das für die anaerobe Fermentierung geeignete Trägermaterial den Fermentierer im Zusammenfluß durchlaufen, daß das zellulosehaltige Trägermaterial und das für die anaerobe Fermentierung geeignete Material an einem Ende des Fermentierers (2) in eine Einspeisungszone (15) genannte Zone eingespeist werden und nach anaerobem Bioabbau auf der entgegengesetzten Seite des Fermentierers (2) von einer Entnahmezone (17) genannten entgegengesetzten Zone des Fermentierers (2) entnommen werden.

6. Verfahren nach Anspruch 5, bei dessen Ausübung der Fermentierer (2) eine im wesentlichen horizontale Achse aufweist, **dadurch gekennzeichnet**, daß das für die Fermentierung geeignete Trägermaterial zurückgeführt wird, daß die Verdichtung des zellulosehaltigen Trägermaterials in der Zone zusätzlicher Verdichtung (16a) des Fermentierers (2) zu einem Niveauanstieg des für die anaerobe Fermentierung geeigneten Materials in der Entnahmezone (17) wie zu einem Absinken des Niveaus dieses Trägermaterials in der Einspeisungszone (15) führt, dergestalt, daß eine Zurückführung ohne Pumpen ermöglicht ist.

7. Verfahren nach Anspruch 6, **dadurch gekennzeichnet**, daß für die Zurückführung des für die anaerobe Fermentierung geeigneten Trägermaterials ein Kanal (29) verwendet wird, der unmittelbar das Oberteil der Entnahmezone (17) und die Einspeisungszone (15) verbindet, und daß der Kanal (29) vorzugsweise in Richtung der Einspeisungzone (15) geneigt ist.

8. Verfahren nach Anspruch 7, **dadurch gekennzeichnet**, daß eine Mengenregelung des für die anaerobe Fermentierung geeigneten Trägermaterials durch ein Steuerventil (31) erfolgt, welches in dem Zurückführungskanal (29) angeordnet ist.

9. Verfahren nach einem der Ansprüche 5 - 8, **dadurch gekennzeichnet**, daß eine Entnahmezone (17) ausgewählt ist, in der die freie Oberfläche des für die Fermentierung geeigneten Trägermaterials mit dem Niveau des Trägermaterials ansteigt.

10. Verfahren nach einem der Ansprüche 5- 9,

dadurch gekennzeichnet, daß der zellulosehaltige Rückstand, der auf der Oberfläche des zu der anaeroben Fermentierung geeigneten Materials schwimmt oder in diesem in der Entnahmezone (17) eingetaucht ist, abgeführt wird, indem der Rückstand nach außen in horizontaler oder vertikaler Richtung mit Hilfe eines mechanischen Mittels versetzt wird, daß das Abführen auch durch Überlauf erfolgen kann, wobei gegebenenfalls der Rückstand mit einem mechanischen Mittel verschoben wird.

11. Verfahren nach einem der Ansprüche 1 - 4, dadurch gekennzeichnet, daß das zellulosehaltige Trägermaterial und das der für die anaerobe Fermentierung geeignete Trägermaterial im Gegenstrom durch den Fermentierer geführt werden.

12. Verfahren nach einem der Ansprüche 1 - 11, dadurch gekennzeichnet, daß das Verdichten einer Charge des zellulosehaltigen Trägermaterials erfolgt, nachdem dieses in die Einspeisungszone (15) eingeführt wurde, aber bevor es zu der Fermentierungszone (16) versetzt wurde, indem die genannte Charge durch die folgende Charge, die in einer Chargierungszone (8) des Fermentierers in Wartestellung ist und in dieser Chargierungszone (8) blockiert ist, belastet wird.

13. Verfahren nach den Ansprüchen 1, 4, 5, 6, 8, 9 und 10, zusammen, dadurch gekennzeichnet, daß das Chargieren der Trägermaterialien, das Vorrücken der Chargen des zellulosehaltigen Trägermaterials, das Zurückführen des für anaerobe Fermentierung geeigneten Materials, der Entzug des zellulosehaltigen Rückstands sowie die Entnahme des für die anaerobe Fermentierung geeigneten Trägermaterials, welches dem gewünschten Abbau unterworfen war, in Abhängigkeit von Größen ferngesteuert werden, welche die Zusammensetzung des für die anaerobe Fermentierung geeigneten Trägermaterials betreffen, welches sich in der Entnahmezone (17) befindet.

14. Einrichtung zur Durchführung des Verfahrens nach einem der Ansprüche 1 - 13, gekennzeichnet durch :
– einen Fermentierer (2);
– ein Mittel zum Einführen des zellulosehaltigen Trägermaterials, welches dem Abbau unterworfen werden soll, in ein Ende des Fermentierers(2);
– ein Mittel zum Verdichten des zellulosehaltigen Trägermaterials, damit dieses verdichtet in den Fermentierer (2) eintritt ;
– eine Einrichtung, um in dem Fermentierer (2) das an dessen einem Ende eingeführte zellulosehaltige Trägermaterial in Richtung des entgegengesetzten Endes fortzubewegen;
– ein Mittel, um die zusätzliche Verdichtung des in den Fermentierer eingeführten zellulosehaltigen Trägermaterials bei Eintritt in die Fermentierungszone (16) des Fermentierers (2) zu bewerkstelligen;
– ein Mittel, um in den Fermentierer (2) an dessen einem Ende das zellulosehaltige Trägermaterial einzuführen, dieses ebenfalls einem Abbau zu unterwerfen und dieses in Richtung auf das entgegengesetzte Ende fortzubewegen;
– einen Sammler für Biogas (28), der in einem oberen Teil des Fermentierers (2) oberhalb dessen Fermentierungszone (16) angeordnet ist;
– einen Kanal (35) zum Abführen des für die Fermentierung geeigneten Trägermaterials im bioabgebauten Zustand; und
– eine Entnahmeeinrichtung des zellulosehaltigen Trägermaterials in bioabgebautem Zustand.

15. Einrichtung nach Anspruch 14, dadurch gekennzeichnet, daß der Fermentierer (2) am Eingang der Fermentierungszone (16) einen geraden, sich verjüngenden Abschnitt darstellt, um die Zone zusätzlicher Verdichtung (16a) zu bilden, wobei die Verjüngung dieses geraden Abschnitts gegebenenfalls nicht in der unteren Zone des Fermentierers (2) durchgeführt ist.

16. Einrichtung nach Anspruch 15, dadurch gekennzeichnet , daß die Zone zusätzlichen Verdichtens (16a) eine kegelstumpfförmig gestaltete Zone ist, die sich in Richtung der Fermentierungszone (16) verjüngt.

17. Einrichtung nach einem der Ansprüche 14 - 16, mit einem Fermentierer (2), in dem das zellulosehaltige Trägermaterial und das für die anaerobe Fermentierung geeignete Material sich in Zusammenfluß fortbewegen können, dadurch gekennzeichnet, daß der Fermentierer (2), der eine langgestreckte Form aufweist und dessen Achse im wesentlichen horizontal angeordnet ist, von einem zum anderen Ende aufeinanderfolgend eine Einspeisungszone (15), eine Fermentierungszone (16) und eine Entnahmezone (17) umfaßt, daß zwei Öffnungen (7, 12) in der Einspeisungszone (15) zum Einführen von Chargen des zellulosehaltigen Trägermaterials und des für die anaerobe Fermentierung geeigneten Materials jeweils vorgesehen sind und daß die Einrichtung (1) gegebenenfalls mit einem Kanal (29) zum Zurückführen durch einfaches Ablaufen der Fermentierungsflüssigkeit von der Entnahmezone (17) zu der Einspeisungszone (15) ausgestattet ist, auf dem gegebenenfalls ein Steuerventil (31) zur Regelung des Rückflusses angeordnet ist.

18. Einrichtung nach Anspruch 17, dadurch gekennzeichnet, daß die Entnahmezone (17) durch eine Wanne (5) begrenzt ist, die eine vertikale Wand (5a) aufweist, in welche rechtwinklig dazu die Wand (3) des Fermentierers (2) mündet, welche die Fermentierungszone (16) begrenzt, und daß der Rückführkanal (29) an die vertikale Wand (5a) in dessen Abschnitt angeschlossen ist, welcher sich oberhalb der Fermentierungszone befindet.

19. Einrichtung nach Anspruch 18, dadurch gekennzeichnet, daß die Wanne (5) eine sich in Richtung auf ihren oberen Abschnitt erweiternde Gestalt aufweist.

20. Einrichtung nach einem der Ansprüche 17 - 19, **dadurch gekennzeichnet**, daß sie oberhalb der Entnahmezone (17) eine Einrichtung (33) aufweist, welche es gestattet, den zellulosehaltigen bioabgebauten Rückstand, der auf der Oberfläche des für die Fermentierung geeigneten Trägermaterials schwimmt oder in dieses eingetaucht ist, in horizontaler Richtung oder in vertikaler Richtung nach außen hin forzubewegen.

21. Einrichtung nach einem der Ansprüche 14 - 20, **dadurch gekennzeichnet**, daß die Einrichtung zum Fortbewegen des zellulosehaltigen Trägermaterials in den Fermentierer (2) einerseits eine Welle (18) umfaßt, welche durch den Fermentierer (2) in Längsrichtung parallel zu dessen Achse hindurchgeführt ist und zur Durchführung einer Hin- und Herbewegung eingerichtet ist, und daß die Welle (n) (18) Zähne (18a) aufweisen, die ein Anhaften des zellulosehaltigen Trägermaterials in der Stoßrichtung und ein Lösen des Trägermaterials in der entgegengesetzten Richtung gestatten, sowie außerdem ein Schuborgan (24 - 26) für dieses Trägermaterial, welches in aufeinanderfolgenden Chargen eingespeist wird, wobei das Schuborgan an der gezahnten Welle oder den gezahnten Wellen (18) befestigt ist.

22. Einrichtung nach einem der Ansprüche 14 - 21, **dadurch gekennzeichnet**, daß sie eine vertikale Rampe (8a) zum Einspeisen des zellulosehaltigen Trägermaterials entsprechend aufeinanderfolgender Chargen aufweist und daß die Rampe (8a) einen Rückhaltefinger (11) für die Chargen vor deren Einspeisung in die Einspeisungszone (15) umfaßt.

23. Einrichtung nach einem der Ansprüche 20 - 22, **dadurch gekennzeichnet**, daß sie eine Fernsteuereinrichtung (48) zum Chargieren der Trägermaterialien, zum Vorschub des zellulosehaltigen Trägermaterials, zum Zurückführen des für die Fermentierung geeigneten Trägermaterials, zur Entnahme des zellulosehaltigen Rückstands und zum Abführen des für die Fermentierung geeigneten Trägermaterials abhängig von den Größem umfaßt, welche das für die Fermentierung geeignete Trägermaterial betreffen, und von einem Element (47) abgegeben werden, welches den Höhenstand der Verschmutzung detektiert und sich in der Entnahmezone (17) befindet.

24. Einrichtung nach einem der Ansprüche 21 - 23, **dadurch gekennzeichnet**, daß die Welle (n) (18) der Einrichtung, um das zellulosehaltige Trägermaterial in dem Fermentierer (2) fortzubewegen, Hohlwellen sind, welche Heizmittel für das Substrat im Innern des Fermentierers einschließen.

## Claims

1. Process which makes it possible to carry out the simultaneous biodegradation, on the one hand, of a cellulosic substrate and, on the other hand, of a substrate suitable for anaerobic fermentation in which the said cellulosic substrate, in a compact state, is capable of being immersed, process according to which each of the aforementioned substrates are made to move inside a fermenter (2), from one end of it to the other, in order to collect the said substrates once they have been subjected to the anaerobic biodegradation, as well as the biogas formed during the fermentation process, characterised in that the cellulosic substrate is subjected to an additional compacting as it enters in the compact state the fermentation zone (16) of the said fermenter (2) which leads to an increase in the density of this substrate moving slowly inside the said fermenter (2) and, consequently, to an increase in its bacteria-fixing potential.

2. Process according to Claim 1, characterised in that the additional compacting of the cellulosic substrate is carried out as it enters the fermentation zone (16) by forcing it to pass through a zone of the fermenter (2) called additional compacting zone (16a), of reduced cross-section.

3. Process according to Claim 2, characterised in that a zone of general frustoconical shape, tapering along the direction of the displacement of the cellulosic substrate, is chosen as additional compacting zone (16a).

4. Process according to one of Claims 1 to 3, characterised in that the substrate suitable for anaerobic fermentation is introduced continuously and the cellulosic substrate sequentially, as a function of the level of purification desired.

5. Process according to one of Claims 1 to 4, characterised in that the cellulosic substrate and the substrate suitable for anaerobic fermentation are made to move concurrently inside the fermenter (2), the cellulosic substrate and the substrate suitable for anaerobic fermentation being introduced at one end of the fermenter (2), in a zone called feed zone (15) and collected, after being subjected to anaerobic biodegradation, at the opposite end of the said fermenter (2), in a zone called extraction zone (17) of the latter.

6. Process according to Claim 5, in which the said fermenter (2) possesses an axis placed substantially horizontally, characterised in that the substrate suitable for fermentation is recycled, the compacting of the cellulosic substrate in the additional compacting zone (16a) of the said fermenter (2) leading to a rise in the level of the substrate suitable for anaerobic fermentation in the said extraction zone (17) as well as to a reduction in the level of this substrate in the feed zone (15), thus enabling a recycling without a pumping means.

7. Process according to Claim 6, characterised in that the recycling of the substrate suitable for anaerobic fermentation is carried out by means of a piping (29) directly connecting the upper part of the extraction zone (17) and the feed zone (15), the said piping (29) being, preferably, inclined in the direction of the feed zone (15).

8. Process according to Claim 7, characterised in that an adjustment of the amount of substrate suitable for anaerobic fermentation which is recycled is ensured by means of a control valve (31) situated on the recycling piping (29).

9. Process according to one of Claims 5 to 8, characterised in that an extraction zone (17) is chosen in which the free surface area of the substrate suitable for anaerobic fermentation increases with the level of the said substrate.

10. Process according to one of Claims 5 to 9, characterised in that the cellulosic residue floating at the surface of, or immersed in, the substrate suitable for anaerobic fermentation in the extraction zone (17) is discharged by displacing the said residue towards the outside in the horizontal direction or in the vertical direction with the aid of a mechanical means, it being also possible for the discharge to be carried out by overflow, where necessary, with the aid of a mechanical means displacing the residue.

11. Process according to one of Claims 1 to 4, characterised in that the cellulosic substrate and the substrate suitable for anaerobic fermentation are made to move countercurrently inside the fermenter.

12. Process according to one of Claims 1 to 11, characterised in that the compacting of a charge of cellulosic substrate is performed after introducing inside the feed zone (15) but before displacing towards the fermentation zone (16), by bringing the next charge waiting in a charging zone (8) of the fermenter (2) and blocked in the said charging zone (8) to bear down on the said charge.

13. Process according to Claims 1, 4, 5, 6, 8, 9 and 10, taken simultaneously, characterised in that the following are remotely controlled: the charging of the substrates, the forward movement of the charges of cellulosic substrate, the recycling of the substrate suitable for anaerobic fermentation, the extraction of the cellulosic residue and the draining of the substrate suitable for anaerobic fermentation, having been subjected to the desired degradation, as a function of data concerning the composition of the substrate suitable for anaerobic fermentation which is in the extraction zone (17).

14. Equipment for implementing the process such as defined in one of Claims 1 to 13, characterised in that it comprises:
– a fermenter (2);
– a means for introducing inside the said fermenter (2), at one end of it, the cellulosic substrate which is to be subjected to a degradation;
– a means for ensuring the compacting of the said cellulosic substrate for it to enter in the compact state the said fermenter (2);
– a device for moving forward inside the said fermenter (2), the cellulosic substrate introduced at one end, in the direction of the opposite end;
– a means for ensuring the additional compacting of the cellulosic substrate introduced in the said fermenter (2), as it enters the fermentation zone (16) of the said fermenter (2);
– a means for introducing inside the said fermenter (2), at one end of it, the cellulosic substrate which must also be subjected to a degradation and for making it flow in the direction of the opposite end;
– a biogas collector (28) placed at the upper part of the said fermenter (2) above the fermentation zone (16);
– a piping (35) for draining the substrate suitable for fermentation, in the biodegraded state; and
– a device for extracting the cellulosic substrate in the biodegraded state.

15. Equipment according to Claim 14, characterised in that the fermenter (2) possesses, at the inlet of the fermentation zone (16), a narrower cross-section in order to constitute the additional compacting zone (16a), the tapering of this cross-section not being optionally implemented in the lower zone of the said fermenter (2).

16. Equipment according to Claim 15, characterised in that the additional compacting zone (16a) is a zone of overall frustoconical shape tapering in the direction of the fermentation zone (16).

17. Equipment according to one of Claims 14 to 16, comprising a fermenter (2) in which the cellulosic substrate and the substrate suitable for anaerobic fermentation are capable of moving concurrently, characterised in that the fermenter (2) which possesses an elongated shape and whose axis is placed substantially horizontally comprises successively, from one end to the other, a feed zone (15), the fermentation zone (16) and an extraction zone (17), two openings (7; 12) being provided in the feed zone (15) for introducing the charges of respectively cellulosic substrate and substrate suitable for anaerobic fermentation, the said equipment (1) being, where necessary, equipped with a piping (29) for recycling by simple flow of the fermentation liquors from the extraction zone (17) towards the feed zone (15) on which is optionally placed a control valve (31) for adjusting the said recycling.

18. Equipment according to Claim 17, characterised in that the extraction zone (17) is delimited by a tank (5) comprising a vertical wall (5a), in which the wall (3) of the fermenter (2) delimiting the fermentation zone (16) opens out perpendicularly, the recycling piping (29) being connected to the said vertical wall (5a), in its section situated above the fermentation zone (16).

19. Equipment according to Claim 18, characterised in that the tank (5) possesses a widening out shape in the direction of its upper part.

20. Equipment according to one of Claims 17 to 19, characterised in that it comprises, above the extraction zone (17), a device (33) which makes it possible to displace towards the outside, in the horizontal direction or in the vertical direction, the biodegraded cellulosic residue floating at the surface of, or immersed in, the substrate suitable for fermentation.

21. Equipment according to one of Claims 14 to 20, characterised in that the device for moving the cellulosic substrate forward inside the fermenter (2) comprises, on the one hand, at least one shaft (18) going across the said fermenter (2) in the longitudinal direction, parallel to its axis, and capable of being driven in a reciprocating movement; the said shaft or shafts (18) comprising indentations (18a) enabling fixing of the cellulosic substrate in the propelling direction and a release of the said substrate in an opposite direction and, on the other hand, an element (24 to 26) for propelling the said substrate, introduced in successive charges, integral with the said indented shaft or shafts (18).

22. Equipment according to one of Claims 14 to 21, characterised in that it comprises a vertical platform (8a) for charging the cellulosic substrate in successive charges, the said platform (8a) comprising a finger (11) for retaining the charges before their introduction inside the feed zone (15).

23. Equipment according to one of Claims 20 to 22, characterised in that it comprises a remote control device (48) for charging the substrates, for moving the cellulosic substrate forward, for recycling the substrate suitable for fermentation, for extracting the cellulosic residue and for draining the substrate suitable for fermentation, as a function of data which concern the substrate suitable for fermentation and arise from an element (47) which detects the level of pollution and which is in the extraction zone (17).

24. Equipment according to one of Claims 21 to 23, characterised in that the shaft or shafts (18) of the device for moving the cellulosic substrate forward inside the fermenter (2) are hollow shafts housing means for heating the said substrate inside the fermenter.

FIG.1

FIG.2

EP 0 241 357 B1

FIG.3

FIG.4

FIG.5

EP 0 241 357 B1